(19) Europäisches Patentamt / European Patent Office / Office européen des brevets

(11) EP 2 416 701 B1

(12) EUROPEAN PATENT SPECIFICATION

(45) Date of publication and mention
of the grant of the patent:
**22.02.2017 Bulletin 2017/08**

(51) Int Cl.:
*A61B 5/00* (2006.01)      *A61B 5/021* (2006.01)
*G06F 17/18* (2006.01)      *A61B 5/024* (2006.01)
*A61B 5/029* (2006.01)

(21) Application number: **10754181.5**

(22) Date of filing: **19.03.2010**

(86) International application number:
**PCT/US2010/028004**

(87) International publication number:
**WO 2010/108110 (23.09.2010 Gazette 2010/38)**

(54) **MONITORING PERIPHERAL DECOUPLING**

ÜBERWACHUNG VON PERIPHERER ENTKOPPLUNG

SURVEILLANCE DU DÉCOUPLAGE PÉRIPHÉRIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.03.2009 US 161942 P**
**09.03.2010 US 720118**

(43) Date of publication of application:
**15.02.2012 Bulletin 2012/07**

(73) Proprietor: **Edwards Lifesciences Corporation Irvine, CA 92614 (US)**

(72) Inventors:
• **HATIB, Feras**
**Irvine, CA 92614 (US)**
• **ROTELIUK, Luchy**
**Irvine, CA 92614 (US)**
• **MCKEOWN, Morgan**
**Irvine, CA 92614 (US)**

(74) Representative: **Eisenführ Speiser**
**Patentanwälte Rechtsanwälte PartGmbB**
**Postfach 31 02 60**
**80102 München (DE)**

(56) References cited:
WO-A1-2008/080194      WO-A2-2009/099833
US-A1- 2002 169 139      US-A1- 2003 093 301
US-A1- 2008 015 451

• DELLA ROCCA G ET AL: "Arterial Pulse Cardiac Output Agreement With Thermodilution in Patients in Hyperdynamic Conditions", JOURNAL OF CARDIOTHORACIC AND VASCULAR ANESTHESIA, SAUNDERS, PHILADELPHIA, PA, US, vol. 22, no. 5, 1 October 2008 (2008-10-01), pages 681-687, XP025464838, ISSN: 1053-0770, DOI: 10.1053/J.JVCA.2008.02.021 [retrieved on 2008-05-14]
• Benjamin Pratt ET AL: "Calculating Arterial Pressure-Based Cardiac Output Using a Novel Measurement and Analysis Method", Biomedical Instrumentation & Technology, 1 January 2007 (2007-01-01), pages 403-411, XP055086642, online Retrieved from the Internet: URL:http://www.researchgate.net/publicatio n/5853603_Calculating_arterial_pressure-ba sed_cardiac_output_using_a_novel_measureme nt_and_analysis_method/file/79e415097ff64b 8867.pdf [retrieved on 2013-11-04]

**Description**

**BACKGROUND**

Indicators such as stroke volume (SV), cardiac output (CO), end-diastolic volume, ejection fraction, stroke volume variation (SW), pulse pressure variation (PPV), and systolic pressure variations (SPV), among others, are important not only for diagnosis of disease, but also for "real-time," i.e., continual, monitoring of clinically significant changes in a subject. For example, health care providers are interested in changes in preload dependence, fluid responsiveness, or volume responsiveness in both human and animal subjects. Few hospitals are therefore without some form of equipment to monitor one or more cardiac indicators in an effort to provide a warning that one or more of the indicated changes are occurring in a subject. Many techniques, including invasive techniques, non-invasive techniques, and combinations thereof, are in use and even more have been proposed in the literature.

WO 2009 / 099833 A1 discloses a method for the detection of a vascular condition in a subject, comprising receiving a signal corresponding to an arterial blood pressure, calculating a cardiovascular parameter using the arterial blood pressure signal based on a set of factors including one ore more parameters effected by the vascular condition, and monitoring the cardiovascular parameter to determine if there is a statistically significant change over time, wherein detection of the statistically significant change in the cardiovascular parameter indicates the vascular condition.

US 2008 / 0015451 A1 discloses a method and apparatus for determining a cardiovascular parameter including receiving an input signal corresponding to an arterial blood pressure measurement over an interval that covers at least one cardiac cycle, determining a propagation time of the input signal, determining at least one statistical moment of the input signal, and determining an estimate of the cardiovascular parameter using the propagation time and the at least one statistical moment.

**SUMMARY**

Devices configured for monitoring central-to-peripheral arterial pressure decoupling in a subject are described. These devices involve sensors for providing arterial pressure waveform data from the subject and software modules for applying a first (decoupled) multivariate statistical model to the arterial pressure waveform data to determine and provide a value for the subject's decoupled arterial tone. The first (decoupled) multivariate statistical model is prepared from a set of arterial pressure waveform data from a group of test subjects that were experiencing central-to-peripheral arterial pressure decoupling. Then the software module applies a second (normal) multivariate statistical model to the arterial pressure waveform data to determine and provide a value for the subject's normal arterial tone. The second (normal) multivariate statistical model is prepared from a set of arterial pressure waveform data from a group of test subjects with normal hemodynamic conditions. Once the subject's decoupled and normal arterial tones are calculated, the values are compared by a calculation module. A difference between the subject's first arterial tone and the subject's second arterial tone greater than a threshold value indicates the subject is experiencing central-to-peripheral arterial pressure decoupling. Similarly, a ratio of the subject's first arterial tone to the subject's second arterial tone greater than a threshold ratio indicates the subject is experiencing central-to-peripheral arterial pressure decoupling. According to the present devices, the difference between the subject's first arterial tone and the subject's second arterial tone is continuously analyzed. In case that the ratio of the subject's first arterial tone to the subject's second arterial tone greater than the threshold ratio may be displayed as a bar graph or a trend graph.

**DESCRIPTION OF DRAWINGS**

Fig. 1 shows simultaneously recorded pressure waveforms in the ascending aorta (Aortic), femoral artery (Femoral), and radial artery (Radial) in a porcine animal model during normal hemodynamic conditions.
Fig. 2 shows simultaneously recorded pressure waveforms in the ascending aorta (Aortic), femoral artery (Femoral), and radial artery (Radial) in a porcine animal model during Endotoxin shock (septic shock) resuscitated with large amounts of fluids and vasopressors.
Fig. 3 shows an example of a complex blood pressure curve over one beat-to-beat heart cycle.
Fig. 4 shows a discrete-time representation of the pressure waveform of Fig. 3.
Fig. 5 shows the area under the systolic portion of the arterial pressure waveform.
Fig. 6 shows the statistical distributions of the area under the systolic phase of the arterial pressure waveform for normal subjects and hyperdynamic subjects.
Fig. 7 shows the duration of the systole for an arterial pressure waveform.
Fig. 8 shows the statistical distribution of the duration of the systole of the arterial pressure waveform for normal subjects and hyperdynamic subjects.

Fig. 9 shows the duration of the systole and the duration of the diastole for an arterial pressure waveform.

Fig. 10 is the statistical distribution of the duration of the diastolic phase for high heart rate subjects in normal hemodynamic conditions (dashed line) and hyperdynamic conditions (thick line)-the distribution for all the patients combined is also shown (thin line).

Fig. 11 is the statistical distribution of the duration of the systolic phase for high heart rate subjects in normal hemodynamic conditions (dashed line) and hyperdynamic conditions (thick line)-the distribution for all the patients combined is also shown (thin line).

Fig. 12 is a block diagram showing the main components of a system to implement the methods described herein.

## DETAILED DESCRIPTION

Devices configured for monitoring central-to-peripheral arterial pressure decoupling, i.e., hyperdynamic conditions are described. These devices are configured to involve the comparison of arterial tones calculated from multivariate statistical models established for both subjects experiencing normal, hemodynamic conditions and subjects experiencing hyperdynamic conditions, during which central-to-peripheral decoupling may occur. The difference between the arterial tones calculated using the two multivariate statistical models can be used to indicate peripheral pressure decoupling when a threshold value is exceeded. These devices are configured to both alert a user to the fact that a subject is experiencing peripheral decoupling and provide accurate arterial tone measurements, which enable the calculation of accurate values for stroke volume and cardiac output, which in turn enable a clinician to appropriately provide treatment to the subject.

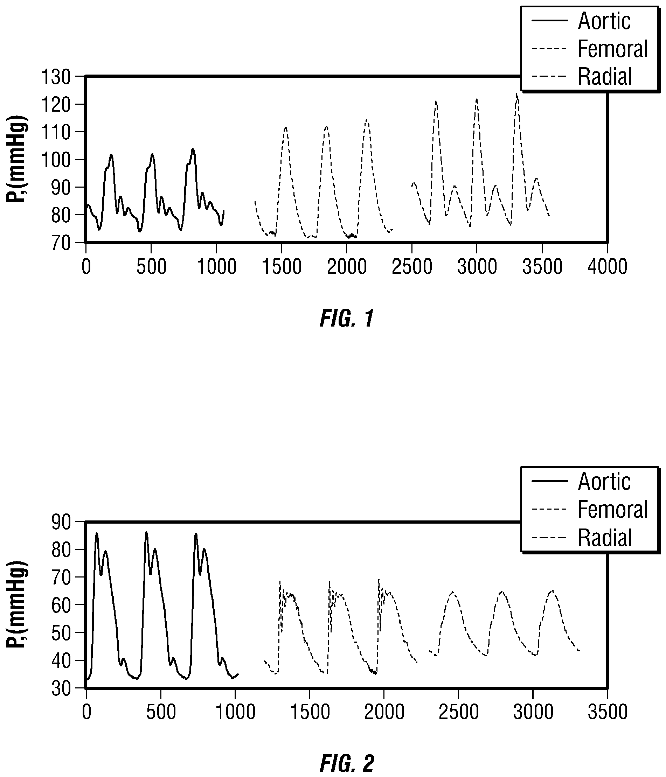

As used herein, the phrases hyperdynamic and vasodilation mean a condition in which peripheral arterial pressure and flow are decoupled from the central aortic pressure and flow, and the term peripheral arteries is intended to mean arteries located away from the heart, e.g., radial, femoral, or brachial arteries. Decoupled arterial pressure means that the normal relationship between peripheral arterial pressure and central aortic pressure is not valid and the peripheral arterial pressure can not be used to determine the central arterial pressure. This also includes conditions in which the peripheral arterial pressure is not proportional or is not a function of the central aortic pressure. Under normal hemodynamic conditions, blood pressure increases the further away from the heart the measurement is taken. Such a pressure increase is shown in Fig. 1, i.e., the amplitude of a pressure wave measured at radial arteries is greater than the pressure measured at the femoral artery, which in turn is greater than the aortic pressure. These differences in pressure are related to wave reflection, i.e., pressure is amplified toward the periphery.

This normal hemodynamic relationship of pressures, i.e., an increase in pressure away from the heart, is often relied upon in medical diagnosis. However, under hyperdynamic/ vasodilation conditions, this relationship can become inverted with the arterial pressure becoming lower than the central aortic pressure. This reversal has been attributed, for example, to arterial tone in the peripheral vessels, which is suggested to impact the wave reflections discussed above. Such a hyperdynamic condition is shown in Fig. 2, i.e., the amplitude of a pressure wave measured at radial arteries is lower than the pressure measured as the femoral artery, which in turn is lower than the aortic pressure. Drugs that dilate small peripheral arteries (e.g., nitrates, ACE inhibitors, and calcium inhibitors) are thought to contribute to hyperdynamic conditions. These types of severe vasodilatory conditions are also often observed in situations right after cardiopulmonary bypass (coronary bypass), in which the radial arterial pressure underestimates the pressure in the aorta. Substantial central to peripheral pressure differences, where the peripheral arterial pressure underestimates the central aortic pressure, are usually observed in patients with severe sepsis who are treated with large amount of fluids and high-dose vasopressors, leading to severe vasodilation. Very similar conditions are also observed in patients with end stage liver disease. As will be well appreciated by those of skill in the art, certain treatments for subjects in normal hemodynamic conditions will be approached differently than for subjects in hyperdynamic conditions. Thus, the presently disclosed devices are configured to detect vasodilation in a subject, if present, and also provide appropriate arterial tone based calculations.

The devices configured for measuring arterial tone in hyperdynamic and non-hyperdynamic subjects described herein are generally configured to implement methods that include the step of providing arterial pressure waveform data from a subject then steps in which the data are analyzed. First the subject's arterial pressure waveform is analyzed to determine the subject's decoupled arterial tone. Next, the subject's arterial pressure waveform is analyzed to determine the subject's normal arterial tone. These steps can be performed in series (in any order) or in parallel. Then the subject's decoupled arterial tone and normal arterial tone are compared. A difference between the subject's decoupled arterial tone and normal arterial tone greater than a threshold value indicates the subject is experiencing central-to-peripheral arterial pressure decoupling. Similarly, a ratio of the subject's decoupled arterial tone to the subject's normal arterial tone greater than a threshold value indicates the subject is experiencing central-to-peripheral arterial pressure decoupling.

In these methods, determining if the subject's peripheral arterial pressure is decoupled from the subject's central aortic pressure involves applying multivariate statistical models to the arterial pressure waveform data. The first (decoupled) multivariate statistical model is prepared from a first set of arterial pressure waveform data from a first group of test subjects that were experiencing decoupling between peripheral arterial pressure and central aortic pressure. The second (normal) multivariate statistical model is prepared from a second set of arterial pressure waveform data from a second group of test subjects that were not experiencing decoupling between peripheral arterial pressure and central aortic pressure. Each multivariate statistical model provides an arterial tone value relative to the two test subject groups.

The multivariate statistical models used herein are based on sets of factors including one or more parameters affected by the subject's vascular condition. Each type of factor used, e.g., pulse beats standard deviation, typically registers a difference between subjects experiencing a particular vascular condition and those not experiencing the condition. This difference, however, is often located along a continuum and a particular subject may have a value between a definite decoupled indication and a definite normal indication or for some reason in that subject the particular factor may appear to be within a normal range even though the subject is experiencing the vascular condition. However, by using multiple factors, i.e., multiple factors impacted by the vascular condition, there will typically be enough positive indications to indicate that a condition is present (or enough negative indications to indicate the condition is not present). Multivariate statistical models as described herein provide the ability to use multiple factors to increase the ability to accurately calculate arterial tone for the two states, i.e., experiencing or not experiencing peripheral decoupling.

The specific number of factors used in a multivariate statistical model will depend on the ability of the individual factors to differentiate between a subject who is experiencing a particular condition and a subject who is not experiencing the particular condition. The number of factors can also be increased to provide a greater level of accuracy to a model. Thus, greater numbers of factors can be used to aid in the precision, accuracy, and/or reproducibility of a model as needed in particular circumstances. Examples of factors that can be used in the models described herein include (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area. Additional factors that can be used with the multivariate statistical models described herein include (o) a parameter based on the shape of the beat-to-beat arterial blood pressure signal and at least one statistical moment of the arterial blood pressure signal having an order of one or greater and (p) a set of anthropometric parameters of the subject. One or more of these factors (or all of these factors) can be used in the multivariate statistical models described herein.

The factors used in the multivariate statistical models described herein are calculated from signals based on arterial blood pressure or signals proportional to arterial blood pressure. The calculation of cardiovascular parameters, such as arterial compliance (arterial tone), is described in U.S. Patent Application Publication US 2005/0124940 A1, filed July 14, 2004. Example of factors and data used in calculating the cardiovascular parameters for use with the methods disclosed herein, including the parameters discussed in U.S. Patent Application Publication US 2005/0124940 A1, are described below.

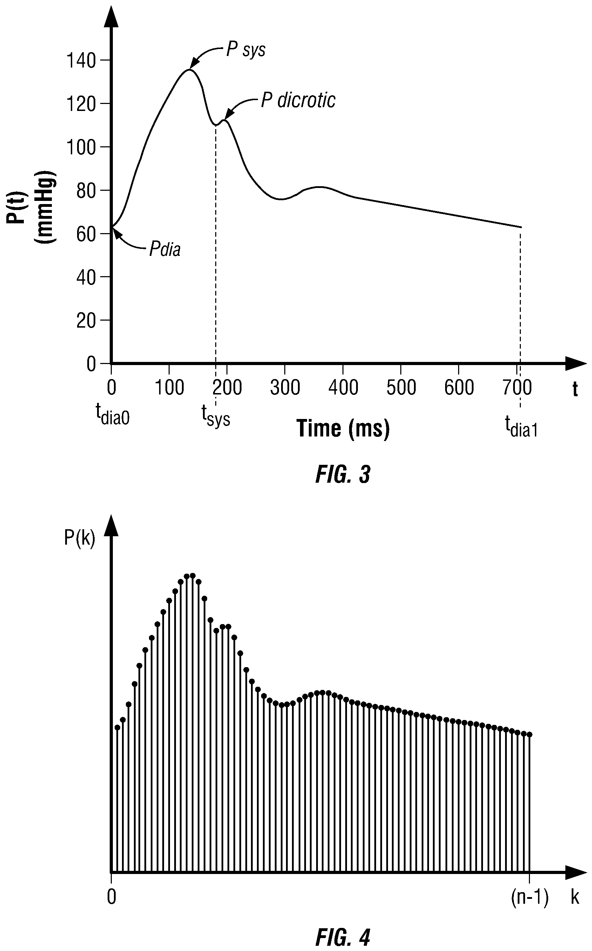

Fig. 3 is an example of an arterial pressure waveform, P(t), taken over a single heart cycle. This heart cycle starts at the point of diastolic pressure $P_{dia}$ at time $t_{dia0}$, through the time $t_{sys}$ of up to systolic pressure $P_{sys}$, to a time $t_{dia1}$ at which the blood pressure once again reaches $P_{dia}$.

Signals useful with the present devices include cardiovascular parameters based on arterial blood pressure or any signal that is proportional to arterial blood pressure, measured at any point in the arterial tree, e.g., radial, femoral, or brachial, either invasively or non-invasively. As used herein, the term arterial pressure waveform data is intended to mean data based on arterial blood pressure or any signal that is proportional to arterial blood pressure. If invasive instruments are used, in particular, catheter-mounted pressure transducers, then any artery is a possible measurement point. Placement of non-invasive transducers will typically be dictated by the instruments themselves, e.g., finger cuffs, upper arm pressure cuffs, and earlobe clamps. Regardless of the specific instrument used, the data obtained will ultimately yield an electric signal corresponding (for example, proportional) to arterial blood pressure.

As illustrated in Fig. 4, analog signals such as arterial blood pressure can be digitized into a sequence of digital values using any standard analog-to-digital converter (ADC). In other words, arterial blood pressure, to $\leq t \leq t_f$, can be converted, using known methods and circuitry, into the digital form P(k), k=0, (n-1), where to and $t_f$ are initial and final times of the measurement interval and n is the number of samples of arterial blood pressure to be included in the calculations, distributed usually evenly over the measurement interval.

To capture relevant data from such digital or digitized signals, consider an ordered collection of m values, that is, a sequence Y(i), where i=1,. .., (m-1). As is well known from the field of statistics, the first four moments $\mu_1$, $\mu_2$, $\mu_3$, and $\mu_4$ of Y(i) can be calculated using known formulas, where $\mu_1$ is the mean (*i.e.*, arithmetic average), $\mu_2 = \sigma^2$ is the variation (*i.e.*, the square of the standard deviation $\sigma$), $\mu_3$ is the skewness, and $\mu_4$ is the kurtosis. Thus:

$$\mu_1 = Y_{avg} = 1/m * \Sigma(Y(i)) \qquad \text{(Formula 1)}$$

$$\mu_2 = \sigma^2 = 1/(m-1) * \Sigma(Y(i)-Y_{avg})^2 \qquad \text{(Formula 2)}$$

$$\mu_3 = 1/(m-1) * \Sigma[(Y(i)-Y_{avg})/\sigma]^3 \qquad \text{(Formula 3)}$$

$$\mu_4 = \sigma/(m-1) * \Sigma[(Y(i)-Y_{avg})/\sigma]^4 \qquad \text{(Formula 4)}$$

In general, the $\beta$-th moment $\mu_p$ can be expressed as:

$$\mu_\beta = 1(m-1)*1/\sigma^\beta*\Sigma[(Y)(i)-Y_{avg})]^\beta \qquad \text{(Formula 5)}$$

 where i=0, ... , (m-1). The discrete-value formulas for the second through fourth moments usually scale by 1/(m-1) instead of 1/m for well-known statistical reasons.

The devices described herein may be configured to implement methods that utilize factors that are a function not only of the four moments of the pressure waveform P(k), but also of a pressure-weighted time vector. Standard deviation $\sigma$ provides one level of shape information in that the greater $\sigma$ is, the more "spread out" the function Y(i) is, *i.e.*, the more it tends to deviate from the mean. Although the standard deviation provides some shape information, its shortcoming can be easily understood by considering the following: the mean and standard deviation will not change if the order in which the values making up the sequence Y(i) is "reversed," that is, Y(i) is reflected about the i=0 axis and shifted so that the value Y(m-1) becomes the first value in time.

Skewness is a measure of lack of symmetry and indicates whether the left or right side of the function Y(i), relative to the statistical mode, is heavier than the other. A positively skewed function rises rapidly, reaches its peak, then falls slowly. The opposite would be true for a negatively skewed function. The point is that the skewness value includes shape information not found in the mean or standard deviation values-in particular, it indicates how rapidly the function initially rises to its peak and then how slowly it decays. Two different functions may have the same mean and standard deviation, but they will then only rarely have the same skewness.

Kurtosis is a measure of whether the function Y(i) is more peaked or flatter than a normal distribution. Thus, a high kurtosis value will indicate a distinct peak near the mean, with a drop thereafter, followed by a heavy "tail." A low kurtosis value will tend to indicate that the function is relatively flat in the region of its peak. A normal distribution has a kurtosis of 3.0; actual kurtosis values are therefore often adjusted by 3.0 so that the values are instead relative to the origin.

An advantage of using the four statistical moments of the beat-to-beat arterial pressure waveform is that the moments are accurate and sensitive mathematical measures of the shape of the beat-to-beat arterial pressure waveform. As arterial compliance and peripheral resistance directly affect the shape of the arterial pressure waveform, the effect of arterial compliance and peripheral resistance could be directly assessed by measuring the shape of the beat-to-beat arterial pressure waveform. The shape sensitive statistical moments of the beat-to-beat arterial pressure waveform along with other arterial pressure parameters described herein could be effectively used to measure the combined effect of vascular compliance and peripheral resistance, *i.e.*, the arterial tone. The arterial tone represents the combined effect of arterial compliance and peripheral resistance and corresponds to the impedance of the well known 2-element electrical analog equivalent model of the Windkessel hemodynamic model, consisting of a capacitive and a resistive component. By measuring arterial tone, several other parameters that are based on arterial tone, such as arterial elasticity, stroke volume, and cardiac output, also could be directly measured. Any of those parameters could be used as factors in the methods the devices described herein are configured to implement.

When the first four moments $\mu_{1P}$, $\mu_{2P}$, $\mu_{3P}$, and $\mu_{4P}$ of the pressure waveform P(k) are calculated and used in a multivariate Boolean or multivariate statistical model, where $\mu_{1P}$ is the mean, $\mu_{2P}$ $P = \sigma_P^2$ is the variation, that is, the square of the standard deviation $\sigma_P$; $\mu_{3P}$ is the skewness, and $\mu_{4P}$ is the kurtosis, where all of these moments are based on the pressure waveform P(k). Formulas 1-4 above may be used to calculate these values after substituting P for Y, k for i, and n for m.

Formula 2 above provides the "textbook" method for computing a standard deviation. Other, more approximate methods may also be used. For example, at least in the context of blood pressure-based measurements, a rough approximation to $\sigma_P$ is to divide by three the difference between the maximum and minimum measured pressure values, and that the maximum or absolute value of the minimum of the first derivative of the P(t) with respect to time is generally proportional to $\sigma_P$.

As Fig. 4 illustrates, at each discrete time k, the corresponding measured pressure will be P(k). The values k and P(k) can be formed into a sequence T(j) that corresponds to a histogram, meaning that each P(k) value is used as a "count" of the corresponding k value. By way of a greatly simplified example, assume that the entire pressure waveform consists of only four measured values P(1)=25, P(2)=50, P(3)=55, and P(4)=35. This could then be represented as a sequence T(j) with 25 ones, 50 twos, 55 threes, and 35 fours:

$$T(j)=1, 1, \ldots, 1, 2, 2, \ldots, 2, 3, 3, \ldots, 3, 4, 4, \ldots, 4$$

This sequence would thus have 25+50+55+35=165 terms.

Moments may be computed for this sequence just as for any other. For example, the mean (first moment) is:

$$\mu_{1T}=(1*25+2*50+3*55+4*35)/165=430/165=2.606 \qquad \text{(Formula 6)}$$

and the standard deviation $\sigma_T$ is the square root of the variation $\mu_{2T}$:

$$SQRT[1/164*25(1-2.61)^2+50(2-2.61)^2+55(3-2.61)^2+35(4-2.61)^2]=0.985$$

The skewness $\mu_{3T}$ and kurtosis $\mu_{4T}$ can be computed by similar substitutions in Formulas 3 and 4:

$$\mu_{3T}=\{1/(164)*(1/\sigma_T^3)\Sigma[P(k)*(k-\mu_{1T})^3]\} \qquad \text{(Formula 7)}$$

$$\mu_{4T}=\{1/(164)*(1/\sigma_T^4)\Sigma[P(k)*(k-\mu_{1T})^4]\} \qquad \text{(Formula 8)}$$

where k=1, ..., (m-1).

As these formulas indicate, this process in effect "weights" each discrete time value k by its corresponding pressure value P(k) before calculating the moments of time. The sequence T(j) has the very useful property that it robustly characterizes the timing distribution of the pressure waveform. Reversing the order of the pressure values P(k) will in almost all cases cause even the mean of T(j) to change, as well as all of the higher-order moments. Moreover, the secondary "hump" that normally occurs at the dicrotic pressure $P_{dicrotic}$ also noticeably affects the value of kurtosis $\mu_{4T}$; in contrast, simply identifying the dicrotic notch in the prior art, such as in the Romano method, requires noisy calculation of at least one derivative.

The pressure weighted moments provide another level of shape information for the beat-to-beat arterial pressure signal, as they are very accurate measures of both the amplitude and the time information of the beat-to-beat arterial pressure signal. Use of the pressure weighted moments in addition to the pressure waveform moments can increase the accuracy of the models described herein.

One cardiovascular parameter useful with the devices described herein is the arterial tone factor $\chi$, which can be used as a cardiovascular parameter by itself or in the calculation of other cardiovascular parameters such as stroke volume or cardiac output. Calculation of the arterial tone $\chi$ may use, e.g., all four of the pressure waveform and pressure-weighted time moments. Additional parameters are included in the computation to take other known characteristics into account, *e.g.*, patient-specific complex pattern of vascular branching. Examples of additional values include, heart rate HR (or period of R-waves), body surface area BSA, or other anthropometric parameters of the subject, a compliance value C(P) calculated using a known method such as described by Langewouters et al. ("The Static Elastic Properties of 45 Human Thoracic and 20 Abnormal Aortas in vitro and the Parameters of a New Model," J. Biomechanics, 17(6):425-435 (1984)), which computes compliance as a polynomial function of the pressure waveform and the patient's age and sex, a parameter based on the shape of the arterial blood pressure signal and at least one statistical moment of the arterial blood pressure signal having an order of one or greater, a parameter based on the area under the systolic portion of the arterial blood pressure signal, a parameter based on the duration of the systole, and a parameter based on the ratio of the duration of the systole to the duration of the diastole.

These last three cardiovascular parameters, *i.e.*, the area under the systolic portion of the arterial blood pressure signal, the duration of the systole, and the ratio of the duration of the systole to the duration of the diastole, are impacted by arterial tone and vascular compliance and, thus, vary, for example, between subjects in normal hemodynamic conditions and subjects in hyperdynamic conditions. Because these three cardiovascular parameters vary between normal and hyperdynamic subjects the devices described herein can use these cardiovascular parameters to detect vasodilation or vasoconstriction in the peripheral arteries of a subject.

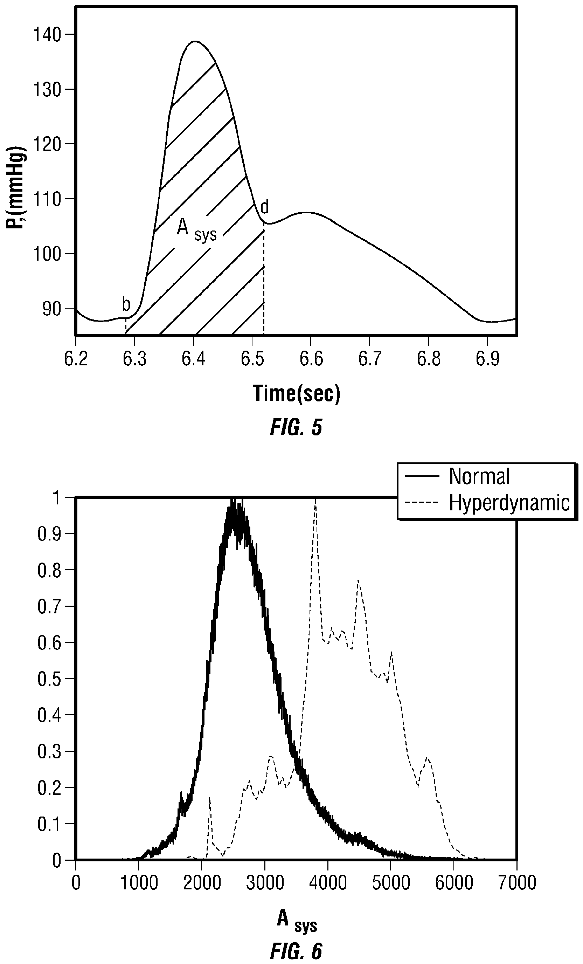

The area under the systolic portion of an arterial pressure waveform ($A_{sys}$) is shown graphically in Fig. 5. The area under the systolic portion of the arterial pressure waveform in an arterial pressure signal is defined as the area under the portion of the waveform starting from the beginning of the beat and ending in the dichrotic notch (from point b to point d on Fig. 5). The area under the systole represents the energy of the arterial pressure signal during systole, which is directly proportional to stroke volume and inversely proportional to arterial compliance. When measured over groups of normal and hyperdynamic patients a shift in $A_{sys}$ can be detected. As shown in Fig. 6, the energy of the arterial pressure signal during systole is higher, for example, in some subjects in hyperdynamic conditions. Those subjects with higher $A_{sys}$ are typically subjects with high cardiac output (CO) and low or normal HR, where the elevated CO is mainly caused by elevated heart contractility, which means that those subjects have increased stroke volume and decreased arterial compliance, which is directly reflected in the energy of the arterial pressure signal during systole. The reflected waves, which are usually very intense during many hyperdynamic conditions, may have also significant contribution to the increased energy of the signal during systole.

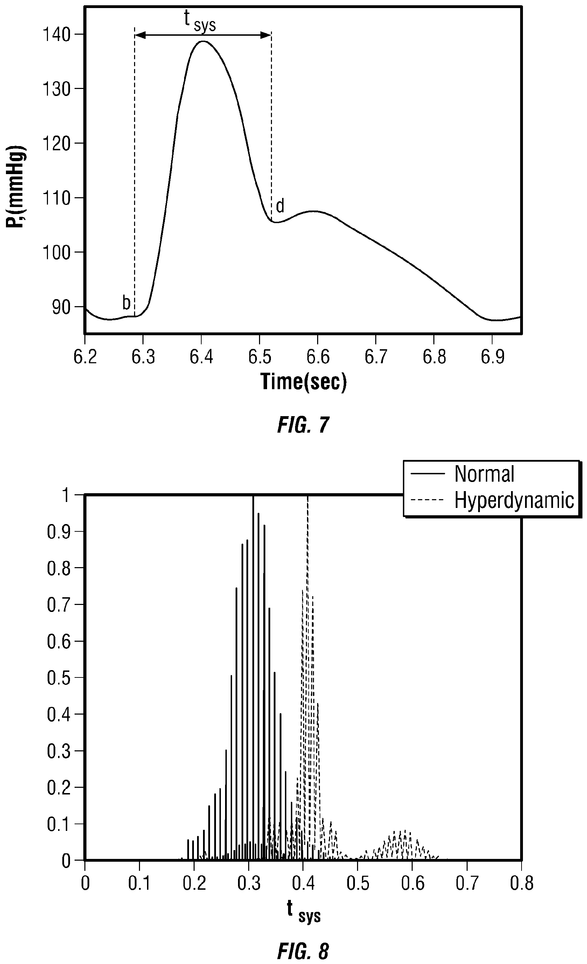

The duration of the systole ($t_{sys}$) is shown graphically in Fig. 7. The duration of the systole in an arterial pressure waveform is defined as the time duration from the beginning of the beat to the dichrotic notch (from point b to point d on Fig. 7). The duration of the systole is directly affected by the arterial compliance and is relatively independent of the changes in peripheral arterial tone, except when large reflect waves are present. As shown on Fig. 8, for example, the duration of the systole in some hyperdynamic subjects is higher than the duration of the systole in normal subjects (data shifted toward higher $t_{sys}$ values). As seen for the systolic energy, the duration of the systole is typically higher in patients with high CO who also have low or normal HR, where the elevated CO is mainly caused by elevated heart contractility and where the contractility may not have been high enough to increase the systolic energy. The increased stroke volume in those patients is partially due to increased contractility and partially due to increased duration of the systole. Reflected waves play a role here as well.

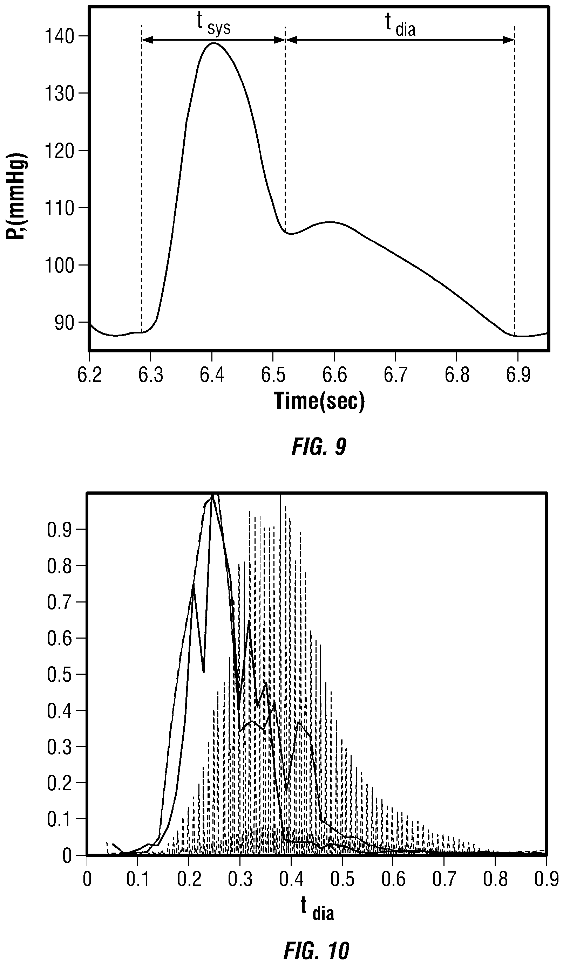

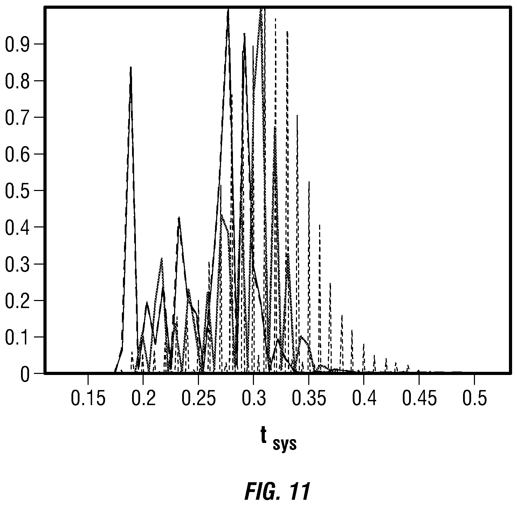

A further parameter that varies, for example, between normal and hyperdynamic subjects is the ratio of the duration of the systole ($t_{sys}$) and the duration of the diastole ($t_{dia}$), as shown graphically in Fig. 9. The duration of the diastole in an arterial pressure waveform is defined as the time duration from the dichrotic notch to the end of the cardiac cycle (from point d to point e on Fig. 9). In some hyperdynamic conditions, the ratio of the durations of the systole and diastole is significantly higher than that observed in normal hemodynamic conditions. This is typically observed in septic shock patients with elevated CO where HR is also high. In these types of conditions, the systole takes over almost the entire cardiac cycle leaving very little time for the diastole before the next cardiac cycle begins. This is shown in Figs. 10 and 11, which show the duration of diastole (Fig. 10) and the duration of systole (Fig. 11) during high HR conditions in septic shock patients and in normal patients. As shown in the figures, high HR patients in normal hemodynamic conditions (dashed line) tend to have low durations of both the systole and the diastole, while high HR patients in septic shock (thick line) tend to have low duration of the diastole but normal or high duration of the systole.

Other parameters based on the arterial tone factor such as, for example, Stroke Volume (SV), Cardiac Output (CO), Arterial Flow, or Arterial Elasticity can be used as factors in the devices described herein. As an example, Stroke Volume (SV) can be calculated as the product of the arterial tone and the standard deviation of the arterial pressure signal:

$$SV = \chi \cdot \sigma_P \qquad \text{(Formula 9)}$$

where:

SV is stroke volume;
$\chi$ is arterial tone; and
$\sigma_p$ is the standard deviation of the arterial pressure

The analog measurement interval, that is, the time window [$t_0$, $t_f$], and thus the discrete sampling interval k=0, ..., (n-1), over which each calculation period is conducted should be small enough so that it does not encompass substantial shifts in the pressure and/or time moments. However, a time window extending longer than one cardiac cycle will provide suitable data. Preferably, the measurement interval is a plurality of cardiac cycles that begin and end at the same point in different cardiac cycles. Using a plurality of cardiac cycles ensures that the mean pressure value used in the calculations of the various higher-order moments will use a mean pressure value $P_{avg}$ that is not biased because of incomplete measurement of a cycle.

Larger sampling windows have the advantage that the effect of perturbations such as those caused by reflections are typically reduced. An appropriate time window can be determined using normal experimental and clinical methods well known to those of skill in the art. Note that it is possible for the time window to coincide with a single heart cycle, in which case mean pressure shifts will not be of concern.

The time window $[t_0, t_f]$ is also adjustable according to drift in $P_{avg}$. For example, if $P_{avg}$ over a given time window differs absolutely or proportionally by more than a threshold amount from the $P_{avg}$ of the previous time window, then the time window can be reduced; in this case stability of $P_{avg}$ is then used to indicate that the time window can be expanded. The time window also can be expanded and contracted based on noise sources, or on a measure of signal-to-noise ratio or variation. Limits are preferably placed on how much the time window is allowed to expand or contract and if such expansion or contraction is allowed at all, then an indication of the time interval is preferably displayed to the user.

The time window does not need to start at any particular point in the cardiac cycle. Thus, to need not be the same as $t_{dia0}$, although this may be a convenient choice in many implementations. Thus, the beginning and end of each measurement interval (*i.e.*, $t_0$ and $t_f$) may be triggered on almost any characteristic of the cardiac cycle, such as at times $t_{dia0}$ or $t_{sys}$, or on non-pressure characteristics such as R waves, etc.

Rather than measure blood pressure directly, any other input signal may be used that is proportional to blood pressure. This means that calibration may be done at any or all of several points in the calculations. For example, if a signal other than arterial blood pressure itself is used as input, then it may be calibrated to blood pressure before its values are used to calculate the various component moments, or afterwards, in which case either the resulting moment values can be scaled. In short, the fact that the cardiovascular parameter may in some cases use a different input signal than a direct measurement of arterial blood pressure does not preclude its ability to generate an accurate compliance estimate.

Creating multivariate statistical models to calculate arterial tone involves several steps. For example, a multiple linear regression response surface methodology can be used to establish the models. The number of terms used in the models can be determined using several numerical approaches to minimize the mean square error between the model output value and arterial tone values determined by alternate methods to which the model is forced. Specifically, a polynomial multivariate fitting function is used to generate the coefficients of the polynomial that give a value of $\chi$ for each set of the arterial pressure waveform parameters, as follows:

$$\chi = \begin{bmatrix} a_1 & a_2 & ... & a_n \end{bmatrix} * \begin{bmatrix} x_1 \\ x_2 \\ ... \\ x_n \end{bmatrix} \qquad \text{(Formula 10)}$$

Where $a_1 ... a_n$ are the coefficients of the polynomial multi-regression model, and $x_1 ... x_n$ are the model's predictor variables. The predictor variables are selected from the factors discussed above that are derived from the arterial pressure waveforms.

Each of the model's predictor variables $\chi_i$ is a predefined combination of the arterial pressure waveform parameters $v_i$ and can be computed as follows:

$$x_i = \prod_m \left( \begin{bmatrix} v_1 & v_2 & ... & v_m \end{bmatrix}^{\wedge \begin{bmatrix} P_{1,1} & ... & P_{1,m} \\ ... & ... & ... \\ P_{n,1} & ... & P_{n,m} \end{bmatrix}} \right) \text{(Formula 11)}$$

The coefficients $v_i$ are different time and frequency domain parameters of the arterial pressure waveform.

As an example, a multivariate statistical model was created using 11 arterial pressure waveform parameters. These parameters were: $v_1$ (standard deviation of the arterial pulse pressure ($\sigma_P$)), $v_2$ (heart rate), $v_3$ (mean arterial pressure ($P_{avg}$)), $v_4$ (pressure weighted standard deviation ($\sigma_T$)), $v_5$ (pressure weighted MAP($\mu_{1T}$)), $v_6$ (skewness of the arterial pulse pressure ($\mu_{3P}$)), $v_7$ (kurtosis of the arterial pulse pressure ($\mu_{4P}$)), $v_8$ (pressure weighted skewness ($\mu_{3T}$)), $v_9$ (pressure weighted kurtosis ($\mu_{4T}$)), $v_{10}$ (pressure dependent Windkessel compliance ($C_W$)), and $v_{11}$ (patient body surface area (BSA)). The coefficients $a_i$ and the exponent matrix "P" can be determined by multivariate least-squares regression using the factor data collected from the subjects. The coefficients and exponent factor are related to the "true" stroke volume, determined through thermodilution, for a population of reference subjects. In this model A and P were established as follows:

$$A = 2.95 \quad -0.43472 \quad 12.384 \quad -143.49 \quad 21.396 \quad -1.3508 \quad 0.029824 \quad -7.3862$$

$$P = \begin{bmatrix} 0 & 0 & 0 & 0 & 0 & 0 & 0 & 0 & 0 & 0 & 1 \\ 0 & 0 & 0 & 0 & 0 & 0 & 0 & 0 & 0 & -1 & 0 \\ 0 & 0 & 2 & 0 & 0 & 0 & 0 & 0 & 0 & 0 & -2 \\ 0 & -2 & 1 & 0 & 0 & 0 & 0 & 0 & 0 & 0 & 0 \\ 0 & -2 & 0 & 0 & 0 & 0 & 0 & 0 & 0 & -1 & 0 \\ 1 & 0 & 0 & 0 & 0 & 0 & -1 & 0 & 0 & 0 & 0 \\ -2 & 0 & 0 & 0 & 0 & 0 & 0 & 0 & 2 & 0 & 0 \\ 0 & -1 & 0 & 0 & 0 & 0 & 0 & 0 & 0 & -1 & -2 \end{bmatrix}$$

Regression was performed in a way to restrain the number of parameters per regression variable to less than three, with each parameter having an order no greater than two. Thus, each row of the matrix P has at most three non-zero terms, with the absolute value of each element of P being at most two. These constraints were used to establish numerical stability and accuracy. The expression for $\chi$ therefore became a second-order curve in eleven-dimensional parameter space. The polynomial expression determined for $\chi$ can be written as follows:

$$x = 2.95 \cdot BSA - 0.43472 \cdot \frac{1}{100 \cdot C_W} + 12.384 \cdot \frac{\left(\frac{1}{100} P_{avg}\right)^2}{BSA^2} - 143.49 \cdot \frac{\frac{1}{100} P_{avg}}{\left(10 \cdot \frac{1}{HR}\right)^2} + 21.396$$

$$\frac{1}{100 \cdot C_W \left(10 \cdot \frac{1}{HR}\right)^2} - 1.3508 \cdot \frac{\frac{1}{10} \sigma_P}{\mu_{4P} + 3} + 0.029824 \cdot \frac{(\mu_{4P} + 3)^2}{\left(\frac{1}{10} \sigma_P\right)^2} -$$

$$7.3862 \cdot \frac{1}{10 \cdot \frac{1}{HR} \cdot 100 \cdot C_W \cdot BSA^2}$$

Thus, a subject's arterial tone can be determined by first creating a model as just described (i.e., determining an approximating function relating a set of clinically derived reference measurements representing blood pressure parameters dependent upon arterial tone, the approximating function being a function of one or more of the parameters described above, and a set of clinically determined reference measurements representing blood pressure parameters dependent upon arterial tone from subjects with normal hemodynamic conditions or subjects experiencing central-to-peripheral arterial pressure decoupling (depending on the model)). Next determining a set of arterial blood pressure parameters from the arterial blood pressure waveform data, the set of arterial blood pressure parameters including the same parameters used to create the multivariate statistical model. Then estimating the subject's normal arterial tone by evaluating the approximating function with the set of arterial blood pressure parameters.

Once multivariate statistical models for the decoupled and normal conditions are established as described above, the models and the devices described herein can be used to continuously calculate a subject's decoupled and normal arterial tones, and monitor difference or ratio changes over time. The difference can be a simple delta ($\Delta$) value between the arterial tones or can, for example, be represented as a percentage difference or change. Similarly, a ratio can be the ratio of the subject's decoupled to normal arterial tones. Regardless of the numerical value calculated to register the difference or ratio between the two arterial tones, a change in this value that exceeds a predetermined threshold value can be used to indicate that the subject is experiencing central-to-peripheral arterial decoupling. Further, values calculated from the arterial tone, e.g., cardiac output, also can be calculated and these differences monitored. As a specific example, the difference between the subject's first arterial tone and the subject's second arterial tone can be calculated as a percentage change in the subject's first arterial tone compared to the subject's second arterial tone. Examples of threshold values using percentage changes include 1% or greater, 2% or greater, 3% or greater, 4% or greater, 5% or greater, 6% or greater, 7% or greater, 8% or greater, 9% or greater, 10% or greater, 15% or greater, 20% or greater, 30% or greater, 40% or greater, and 50% or greater. Similarly, example of threshold values using a ratio of the subject's decoupled arterial tone to the subject's normal arterial tone include 1.01 or greater, 1.02 or greater, 1.03 or greater, 1.04 or greater, 1.05 or greater, 1.06 or greater, 1.07 or greater, 1.08 or greater, 1.09 or greater, 1.10 or greater, 1.15 or greater, 1.20 or greater, 1.30 or greater, 1.40 or greater, and 1.50 or greater. The device can further be configured to alert a user when decoupling or hyperdynamic conditions are determined. Such an alert can be a notice published on a graphical user interface or a sound.

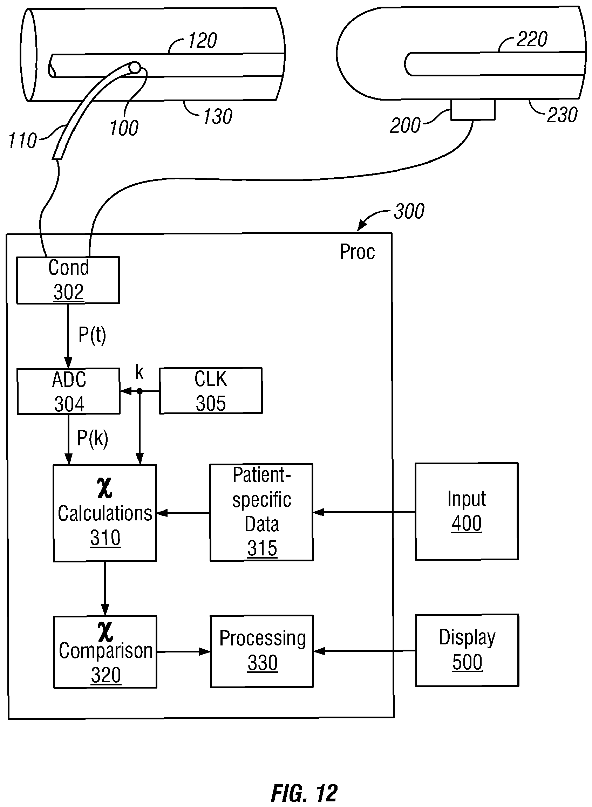

Fig. 12 shows the main components of a system that implements the methods described herein for monitoring central-to-peripheral decoupling in a subject. The methods may be implemented within an existing patient-monitoring device, or it may be implemented as a dedicated monitor. As is mentioned above, pressure, or some other input signal proportional to pressure, may be sensed in either or, indeed, both, of two ways: invasively and non-invasively. For convenience, the system is described as measuring arterial blood pressure as opposed to some other input signal that is converted to pressure.

Fig. 12 shows both types of pressure sensing for the sake of completeness. In most practical applications of the methods described herein, either one or several variations will typically be implemented. In invasive applications of the methods described herein, a conventional pressure sensor 100 is mounted on a catheter 110, which is inserted in an artery 120 of a portion 130 of the body of a human or animal patient. The artery 120 is any artery in the arterial system, such as, for example, the femoral, radial or brachial artery. In the non-invasive applications of the methods described herein, a conventional pressure sensor 200, such as a photo-plethysmographic blood pressure probe, is mounted externally in any conventional manner, for example using a cuff around a finger 230 or a transducer mounted on the wrist of the patient. Fig. 12 schematically shows both types.

The signals from the sensors 100, 200 are passed via any known connectors as inputs to a processing system 300, which includes one or more processors and other supporting hardware and system software (not shown) usually included to process signals and execute code. The methods described herein may be implemented using a modified, standard, personal computer, or may be incorporated into a larger, specialized monitoring system. For use with the methods described herein, the processing system 300 also may include, or is connected to, conditioning circuitry 302 which performs normal signal processing tasks such as amplification, filtering, or ranging, as needed. The conditioned, sensed input pressure signal P(t) is then converted to digital form by a conventional analog-to-digital converter ADC 304, which has or takes its time reference from a clock circuit 305. As is well understood, the sampling frequency of the ADC 304 should be chosen with regard to the Nyquist criterion so as to avoid aliasing of the pressure signal (this procedure is very well known in the art of digital signal processing). The output from the ADC 304 will be the discrete pressure signal P(k), whose values may be stored in conventional memory circuitry (not shown).

The values P(k) are passed to or accessed from memory by a software module 310 comprising computer-executable code for implementing the multivariate statistical models to determine the subject's decoupled and hemodynamic arterial tones. The design of such a software module 310 will be straight forward to one of skill in the art of computer programming.

If used, patient-specific data such as age, height, weight, BSA, etc., is stored in a memory region 315, which may also store other predetermined parameters such as threshold or threshold range values. These values may be entered using any known input device 400 in the conventional manner.

Comparison of the arterial tones is done in module 320. Calculation module 320 includes computer-executable code and take as inputs the output of module 310, then performs the chosen arterial tone calculations.

As illustrated by Fig. 12, the results may be passed to further modules (330) for additional processing and ultimately displayed on a conventional display or recording device 500 for presentation to and interpretation by a user. As with the input device 400, the display 500 will typically be the same as is used by the processing system for other purposes.

For each of the methods described herein, when decoupling is detected, a user can be notified. The user can be notified of the decoupling by publishing a notice on display 500 or another graphical user interface device. Further, a sound can be used to notify the user of the decoupling. Both visual and auditory signals can be used.

Exemplary embodiments of the present invention have been described above with reference to a block diagram of methods, apparatuses, and computer program products. One of skill will understand that each block of the block diagram, and combinations of blocks in the block diagram, respectively, can be implemented by various means including computer program instructions. These computer program instructions may be loaded onto a general purpose computer, special purpose computer, or other programmable data processing apparatus to produce a machine, such that the instructions which execute on the computer or other programmable data processing apparatus create a means for implementing the functions specified in the blocks.

The methods described herein further relate to computer program instructions that may be stored in a computer-readable memory that can direct a computer or other programmable data processing apparatus, such as in a processor or processing system (shown as 300 in Fig. 12), to function in a particular manner, such that the instructions stored in the computer-readable memory produce an article of manufacture including computer-readable instructions for implementing the function specified in the blocks illustrated in Fig. 12. The computer program instructions may also be loaded onto a computer, the processing system 300, or other programmable data processing apparatus to cause a series of operational steps to be performed on the computer, the processing system 300, or other programmable apparatus to produce a computer-implemented process such that the instructions that execute on the computer or other programmable apparatus provide steps for implementing the functions specified in the blocks. Moreover, the various software modules 310, 320, and 330 used to perform the various calculations and perform related method steps described herein also can be stored as computer-executable instructions on a computer-readable medium in order to allow the methods to be loaded into and executed by different processing systems.

Accordingly, blocks of the block diagram support combinations of means for performing the specified functions, combinations of steps for performing the specified functions, and program instruction means for performing the specified functions. One of skill will understand that each block of the block diagram, and combinations of blocks in the block diagram, can be implemented by special purpose hardware-based computer systems that perform the specified functions or steps, or combinations of special purpose hardware and computer instructions.

The present invention is not limited in scope by the embodiments disclosed herein which are intended as illustrations of a few aspects of the invention and any embodiments which are functionally equivalent are within the scope of this invention, which is defined by the appended claims. The term "comprising" and variations thereof as used herein is used synonymously with the term "including" and variations thereof and are open, non-limiting terms.

**Claims**

1. A device configured for monitoring central-to-peripheral arterial pressure decoupling in a subject comprising:

    a sensor (100, 200) for providing arterial pressure waveform data from the subject;
    a processing system (300) for executing code;
    a software module (310) comprising computer-executable code for applying a first multivariate statistical model to the arterial pressure waveform data to determine the subject's first arterial tone, the first multivariate statistical model being prepared from a set of arterial pressure waveform data from a group of test subjects that were experiencing central-to-peripheral arterial pressure decoupling, the first multivariate statistical model providing a value for the subject's first arterial tone,
    wherein the software module (310) further comprises computer-executable code for applying a second multivariate statistical model to the arterial pressure waveform data to determine the subject's normal arterial tone, the second multivariate statistical model being prepared from a set of arterial pressure waveform data from a group of test subjects with normal hemodynamic conditions, the second multivariate statistical model providing a value for the subject's second arterial tone; and
    a calculation module (320) comprising computer-executable code for comparing the subject's first arterial tone to the subject's second arterial tone;
    wherein the processing system (300) determines central-to-peripheral arterial pressure decoupling when a difference between the subject's first arterial tone and the subject's second arterial tone greater than a threshold value indicates that the subject is experiencing central-to-peripheral arterial pressure decoupling.

2. A device configured for monitoring central-to-peripheral arterial pressure decoupling in a subject comprising:

a sensor (100, 200) for providing arterial pressure waveform data from the subject;
a processing system (300) for executing code;
a software module (310) comprising computer-executable code for applying a first multivariate statistical model to the arterial pressure waveform data to determine the subject's first arterial tone, the first multivariate statistical model being prepared from a set of arterial pressure waveform data from a group of test subjects that were experiencing central-to-peripheral arterial pressure decoupling, the first multivariate statistical model providing a value for the subject's first arterial tone;
wherein the software module (310) further comprises computer-executable code for applying a second multivariate statistical model to the arterial pressure waveform data to determine the subject's normal arterial tone, the second multivariate statistical model being prepared from a set of arterial pressure waveform data from a group of test subjects with normal hemodynamic conditions, the second multivariate statistical model providing a value for the subject's second arterial tone; and
a calculation module (320) comprising computer-executable code for comparing the subject's first arterial tone to the subject's second arterial tone,
wherein the processing system (300) determines central-to-peripheral arterial pressure decoupling when a ratio of the subject's first arterial tone to the subject's second arterial tone greater than a threshold ratio indicates that the subject is experiencing central-to-peripheral arterial pressure decoupling.

3. The device of one of claims 1 or 2, the calculation module (320) further configured for using the subject's first arterial tone and the subject's second arterial tone to calculate a subject's first cardiac output and a subject's second cardiac output, wherein a difference between the subject's first cardiac output and the subject's second cardiac output greater than a threshold value indicates the subject is experiencing central-to-peripheral arterial pressure decoupling.

4. The device of one of claims 1 or 2, wherein the calculation module (320) is configured to calculate the difference between the subject's first arterial tone and the subject's second arterial tone as a percentage change in the subject's first arterial tone compared to the subject's second arterial tone.

5. The device of one of claims 1 or 2, wherein the multivariate statistical model is based on a set of factors including one or more parameters affected by the vascular condition.

6. The device of one of claims 1 or 2, wherein the multivariate statistical model is based on a set of factors including one or more parameters selected from the group consisting of (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area.

7. The device of one of claims 1 or 2, wherein the multivariate statistical model is based on a set of factors including (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area.

8. The device of claim1, wherein the threshold value is 1 % or greater.

9. The device of claim 1, further including a graphical user interface (500) configured to display the difference between the subject's first arterial tone and the subject's second arterial tone greater than the threshold value.

10. The device of claim 9, the graphical user interface (500) configured to display the difference between the subject's first arterial tone and the subject's second arterial greater than the threshold value as a bar graph or a trend graph.

11. The device of claim 2, further including a graphical user interface (500) configured to display the ratio of the subject's first arterial tone to the subject's second arterial tone greater than a threshold ratio.

12. The device of claim 1, further comprising processing means (330) for alerting a user when the difference between the subject's first arterial tone and the subject's second arterial tone is greater than the threshold value.

13. The device of claim 2, further comprising processing means (330) for alerting a user when the ratio of the subject's first arterial tone to the subject's second arterial tone is greater than the threshold ratio.

14. The device of one of claims 1 or 2, wherein the software module (310) is configured to determine the subject's first arterial tone by applying the first multivariate statistical model created using the following steps:

determining an approximating function relating a set of clinically derived reference measurements representing blood pressure parameters dependent upon arterial tone, the approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area, and a set of clinically determined reference measurements representing blood pressure parameters dependent upon arterial tone from subjects experiencing central-to-peripheral arterial pressure decoupling;
determining a set of arterial blood pressure parameters from the arterial blood pressure waveform data, the set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and
estimating the subject's first arterial tone by evaluating the approximating function with the set of arterial blood pressure parameters.

15. The device of one of claims 1 or 2, wherein the software module (310) is configured to determine the subject's normal arterial by applying the second multivariate statistical model created using the following steps:

determining an approximating function relating a set of clinically derived reference measurements representing blood pressure parameters dependent upon arterial tone, the approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area, and a set of clinically determined reference measurements representing blood pressure parameters dependent upon arterial tone from subjects with normal hemodynamic conditions;

determining a set of arterial blood pressure parameters from the arterial blood pressure waveform data, the set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and

estimating the subject's normal arterial tone by evaluating the approximating function with the set of arterial blood pressure parameters.

## Patentansprüche

1. Vorrichtung, gestaltet zum Überwachen einer Zentrum-zu-Peripherie-Arteriendruckentkopplung in einem Probanden, umfassend:

einen Sensor (100, 200) zum Bereitstellen von Arteriendruck-Signalform-Daten von dem Probanden;
ein Verarbeitungssystem (300) zum Ausführen eines Codes;
ein Softwaremodul (310), umfassend computerausführbaren Code zum Anwenden eines ersten multivariaten statistischen Modells auf die Arteriendruck-Signalform-Daten zur Bestimmung des ersten Arterientonus des Probanden, wobei das erste multivariate statistische Modell aus einem Satz Arteriendruck-Signalform-Daten von einer Gruppe von Probanden erstellt ist, die Zentrum-zu-Peripherie-Arteriendruckentkopplung erfahren, wobei das erste multivariate statistische Modell einen Wert für den ersten Arterientonus des Probanden bereitstellt,
wobei das Softwaremodul (310) ferner computerausführbaren Code zum Anwenden eines zweiten multivariaten statistischen Modells auf die Arteriendruck-Signalform-Daten umfasst, um den normalen Arterientonus des Probanden zu bestimmen, wobei das zweite multivariate statistische Modell aus einem Satz Arteriendruck-Signalform-Daten von einer Gruppe von Probanden mit normalen hämodynamischen Bedingungen erstellt ist, wobei das zweite multivariate statistische Modell einen Wert für den zweiten Arterientonus des Probanden bereitstellt; und
ein Berechnungsmodul (320), umfassend computerausführbaren Code zum Vergleichen des ersten Arterientonus des Probanden mit dem zweiten Arterientonus des Probanden;
wobei das Verarbeitungssystem (300) eine Zentrum-zu-Peripherie-Arteriendruckentkopplung feststellt, wenn eine Differenz zwischen dem ersten Arterientonus des Probanden und dem zweiten Arterientonus des Probanden, die größer ist als ein Schwellenwert, anzeigt, dass der Proband eine Zentrum-zu-Peripherie-Arteriendruckentkopplung erfährt.

2. Vorrichtung, gestaltet zum Überwachen einer Zentrum-zu-Peripherie-Arteriendruckentkopplung in einem Probanden, umfassend:

einen Sensor (100, 200) zum Bereitstellen von Arteriendruck-Signalform-Daten von dem Probanden;
ein Verarbeitungssystem (300) zum Ausführen eines Codes;
ein Softwaremodul (310), umfassend computerausführbaren Code zum Anwenden eines ersten multivariaten statistischen Modells auf die Arteriendruck-Signalform-Daten zur Bestimmung des ersten Arterientonus des Probanden, wobei das erste multivariate statistische Modell aus einem Satz Arteriendruck-Signalform-Daten von einer Gruppe von Probanden erstellt ist, die Zentrum-zu-Peripherie-Arteriendruckentkopplung erfahren, wobei das erste multivariate statistische Modell einen Wert für den ersten Arterientonus des Probanden bereitstellt;
wobei das Softwaremodul (310) ferner computerausführbaren Code zum Anwenden eines zweiten multivariaten statistischen Modells auf die Arteriendruck-Signalform-Daten umfasst, um den normalen Arterientonus des Probanden zu bestimmen, wobei das zweite multivariate statistische Modell aus einem Satz Arteriendruck-Signalform-Daten von einer Gruppe von Probanden mit normalen hämodynamischen Bedingungen erstellt ist, wobei das zweite multivariate statistische Modell einen Wert für den zweiten Arterientonus des Probanden bereitstellt; und
ein Berechnungsmodul (320), umfassend computerausführbaren Code zum Vergleichen des ersten Arterientonus des Probanden mit dem zweiten Arterientonus des Probanden,
wobei das Verarbeitungssystem (300) eine Zentrum-zu-Peripherie-Arteriendruckentkopplung feststellt, wenn ein Verhältnis des ersten Arterientonus des Probanden zum zweiten Arterientonus des Probanden, das größer ist als ein Verhältnis-Schwellenwert, anzeigt, dass der Proband eine Zentrum-zu-Peripherie-Arteriendruckentkopplung erfährt.

3. Vorrichtung nach einem der Ansprüche 1 oder 2, wobei das Berechnungsmodul (320) ferner gestaltet ist, den ersten Arterientonus des Probanden und den zweiten Arterientonus des Probanden zu verwenden, um eine erste Herzleistung des Probanden und eine zweite Herzleistung des Probanden zu berechnen, wobei eine Differenz zwischen der ersten Herzleistung des Probanden und der zweiten Herzleistung des Probanden, die größer ist als ein Schwellenwert, anzeigt, dass der Proband eine Zentrum-zu-Peripherie-Arteriendruckentkopplung erfährt.

4. Vorrichtung nach einem der Ansprüche 1 oder 2, wobei das Berechnungsmodul (320) gestaltet ist, die Differenz zwischen dem ersten Arterientonus des Probanden und dem zweiten Arterientonus des Probanden als eine prozentuale Änderung des ersten Arterientonus des Probanden im Vergleich zum zweiten Arterientonus des Probanden zu berechnen.

5. Vorrichtung nach einem der Ansprüche 1 oder 2, wobei das multivariate statistische Modell auf einem Satz von Faktoren beruht, die einen oder mehrere Parameter umfassen, die durch den Gefäßzustand beeinflusst werden.

6. Vorrichtung nach einem der Ansprüche 1 oder 2, wobei das multivariate statistische Modell auf einem Satz von Faktoren beruht, die einen oder mehrere Parameter umfassen, die aus der Gruppe ausgewählt sind, die umfasst: (a) einen Parameter, der auf der Standardabweichung der Arteriendruck-Signalform-Daten beruht, (b) einen Parameter, der auf der Herzfrequenz des Probanden beruht, (c) einen Parameter, der auf der Fläche unter dem systolischen Abschnitt des arteriellen Blutdrucksignals beruht, (d) einen Parameter, der auf der Dauer der Systole beruht, (e) einen Parameter, der auf dem Verhältnis der Dauer der Systole zur Dauer der Diastole beruht, (f) einen Parameter, der auf dem mittleren arteriellen Blutdruck eines Satzes von Arteriendruck-Signalform-Daten beruht, (g) einen Parameter, der auf der nach Druck gewichteten Standardabweichung eines Satzes von Arteriendruck-Signalform-Daten beruht, (h) einen Parameter, der auf dem nach Druck gewichteten Mittelwert eines Satzes von Arteriendruck-Signalform-Daten beruht, (i) einen Parameter, der auf den Schiefewerten der arteriellen Pulsschläge eines Satzes von Arteriendruck-Signalform-Daten beruht, (j) einen Parameter, der auf den Kurtosiswerten der arteriellen Pulsschläge eines Satzes von Arteriendruck-Signalform-Daten beruht, (k) einen Parameter, der auf der nach Druck gewichteten Schiefe eines Satzes von Arteriendruck-Signalform-Daten beruht, (l) einen Parameter, der auf der nach Druck gewichteten Kurtosis eines Satzes von Arteriendruck-Signalform-Daten beruht, (m) einen Parameter, der auf der druckabhängigen Windkessel-Compliance eines Satzes von Arteriendruck-Signalform-Daten beruht, und (n) einen Parameter, der auf der Körperoberfläche des Probanden beruht.

**7.** Vorrichtung nach einem der Ansprüche 1 oder 2, wobei das multivariate statistische Modell auf einem Satz von Faktoren beruht, umfassend: (a) einen Parameter, der auf der Standardabweichung der Arteriendruck-Signalform-Daten beruht, (b) einen Parameter, der auf der Herzfrequenz des Probanden beruht, (c) einen Parameter, der auf der Fläche unter dem systolischen Abschnitt des arteriellen Blutdrucksignals beruht, (d) einen Parameter, der auf der Dauer der Systole beruht, (e) einen Parameter, der auf dem Verhältnis der Dauer der Systole zur Dauer der Diastole beruht, (f) einen Parameter, der auf dem mittleren arteriellen Blutdruck eines Satzes von Arteriendruck-Signalform-Daten beruht, (g) einen Parameter, der auf der nach Druck gewichteten Standardabweichung eines Satzes von Arteriendruck-Signalform-Daten beruht, (h) einen Parameter, der auf dem nach Druck gewichteten Mittelwert eines Satzes von Arteriendruck-Signalform-Daten beruht, (i) einen Parameter, der auf den Schiefewerten der arteriellen Pulsschläge eines Satzes von Arteriendruck-Signalform-Daten beruht, (j) einen Parameter, der auf den Kurtosiswerten der arteriellen Pulsschläge eines Satzes von Arteriendruck-Signalform-Daten beruht, (k) einen Parameter, der auf der nach Druck gewichteten Schiefe eines Satzes von Arteriendruck-Signalform-Daten beruht, (l) einen Parameter, der auf der nach Druck gewichteten Kurtosis eines Satzes von Arteriendruck-Signalform-Daten beruht, (m) einen Parameter, der auf der druckabhängigen Windkessel-Compliance eines Satzes von Arteriendruck-Signalform-Daten beruht, und (n) einen Parameter, der auf der Körperoberfläche des Probanden beruht.

**8.** Vorrichtung nach Anspruch 1, wobei der Schwellenwert 1 % oder größer ist.

**9.** Vorrichtung nach Anspruch 1, ferner umfassend eine grafische Benutzerschnittstelle (500), die gestaltet ist, die Differenz zwischen dem ersten Arterientonus des Probanden und dem zweiten Arterientonus des Probanden anzuzeigen, die größer als der Schwellenwert ist.

**10.** Vorrichtung nach Anspruch 9, wobei die grafische Benutzerschnittstelle (500) gestaltet ist, die Differenz zwischen dem ersten Arterientonus des Probanden und dem zweiten Arterientonus des Probanden, die größer als der Schwellenwert ist, als Balkendiagramm oder Trenddarstellung anzuzeigen.

**11.** Vorrichtung nach Anspruch 2, ferner umfassend eine grafische Benutzerschnittstelle (500), die gestaltet ist, das Verhältnis des ersten Arterientonus des Probanden zum zweiten Arterientonus des Probanden anzuzeigen, das größer als ein Verhältnis-Schwellenwert ist.

**12.** Vorrichtung nach Anspruch 1, ferner umfassend Verarbeitungseinrichtungen (330) zum Alarmieren eines Benutzers, wenn die Differenz zwischen dem ersten Arterientonus des Probanden und dem zweiten Arterientonus des Probanden größer als der Schwellenwert ist.

**13.** Vorrichtung nach Anspruch 2, ferner umfassend Verarbeitungseinrichtungen (330) zum Alarmieren eines Benutzers, wenn das Verhältnis des ersten Arterientonus des Probanden zum zweiten Arterientonus des Probanden größer als der Verhältnis-Schwellenwert ist.

**14.** Vorrichtung nach einem der Ansprüche 1 oder 2, wobei das Softwaremodul (310) gestaltet ist, den ersten Arterientonus des Probanden zu bestimmen, indem das erste multivariate statistische Modell angewendet wird, das unter Verwendung folgender Schritte erzeugt wird:

Bestimmen einer Approximationsfunktion, die einen Satz von klinisch hergeleiteten Referenzmessungen, die Blutdruckparameter repräsentieren, die vom Arterientonus abhängen, wobei die Approximationsfunktion eine Funktion ist von mindestens: (a) einem Parameter, der auf der Standardabweichung der Arteriendruck-Signalform-Daten beruht, (b) einem Parameter, der auf der Herzfrequenz des Probanden beruht, (c) einem Parameter, der auf der Fläche unter dem systolischen Abschnitt des arteriellen Blutdrucksignals beruht, (d) einem Parameter, der auf der Dauer der Systole beruht, (e) einem Parameter, der auf dem Verhältnis der Dauer der Systole zur Dauer der Diastole beruht, (f) einem Parameter, der auf dem mittleren arteriellen Blutdruck eines Satzes von Arteriendruck-Signalform-Daten beruht, (g) einem Parameter, der auf der nach Druck gewichteten Standardabweichung eines Satzes von Arteriendruck-Signalform-Daten beruht, (h) einem Parameter, der auf dem nach Druck gewichteten Mittelwert eines Satzes von Arteriendruck-Signalform-Daten beruht, (i) einem Parameter, der auf den Schiefewerten der arteriellen Pulsschläge eines Satzes von Arteriendruck-Signalform-Daten beruht, (j) einem Parameter, der auf den Kurtosiswerten der arteriellen Pulsschläge eines Satzes von Arteriendruck-Signalform-Daten beruht, (k) einem Parameter, der auf der nach Druck gewichteten Schiefe eines Satzes von Arteriendruck-Signalform-Daten beruht, (l) einem Parameter, der auf der nach Druck gewichteten Kurtosis eines Satzes von Arteriendruck-Signalform-Daten beruht, (m) einem Parameter, der auf der druckabhängigen Windkessel-Compliance eines Satzes von Arteriendruck-Signalform-Daten beruht, und (n) einem Parameter, der auf der Körperoberfläche des Probanden beruht, und einen Satz von klinisch bestimmten Referenzmessungen, die Blutdruckparameter repräsentieren, die vom Arterientonus von Probanden abhängen, die eine Zentrum-zu-Peripherie-Arteriendruckentkopplung erfahren, verknüpft;

Bestimmen eines Satzes von arteriellen Blutdruck-Parametern aus den arteriellen Blutdruck-Signalform-Daten, wobei der Satz von arteriellen Blutdruck-Parametern mindestens umfasst: (a) einen Parameter, der auf der Standardabweichung der Arteriendruck-Signalform-Daten beruht, (b) einen Parameter, der auf der Herzfrequenz des Probanden beruht, (c) einen Parameter, der auf der Fläche unter dem systolischen Abschnitt des arteriellen Blutdrucksignals beruht, (d) einen Parameter, der auf der Dauer der Systole beruht, (e) einen Parameter, der auf dem Verhältnis der Dauer der Systole zur Dauer der Diastole beruht, (f) einen Parameter, der auf dem mittleren arteriellen Blutdruck eines Satzes von Arteriendruck-Signalform-Daten beruht, (g) einen Parameter, der auf der nach Druck gewichteten Standardabweichung eines Satzes von Arteriendruck-Signalform-Daten beruht, (h) einen Parameter, der auf dem nach Druck gewichteten Mittelwert eines Satzes von Arteriendruck-Signalform-Daten beruht, (i) einen Parameter, der auf den Schiefewerten der arteriellen Pulsschläge eines Satzes von Arteriendruck-Signalform-Daten beruht, (j) einen Parameter, der auf den Kurtosiswerten der arteriellen Pulsschläge eines Satzes von Arteriendruck-Signalform-Daten beruht, (k) einen Parameter, der auf der nach Druck gewichteten Schiefe eines Satzes von Arteriendruck-Signalform-Daten beruht, (l) einen Parameter, der auf der nach Druck gewichteten Kurtosis eines Satzes von Arteriendruck-Signalform-Daten beruht, (m) einen Parameter, der auf der druckabhängigen Windkessel-Compliance eines Satzes von Arteriendruck-Signalform-Daten beruht, und (n) einen Parameter, der auf der Körperoberfläche des Probanden beruht; und

Berechnen des ersten Arterientonus des Probanden durch Auswerten der Approximationsfunktion mit dem Satz von arteriellen Blutdruck-Parametern.

15. Vorrichtung nach einem der Ansprüche 1 oder 2, wobei das Softwaremodul (310) gestaltet ist, den normalen Arterientonus des Probanden zu bestimmen, indem das zweite multivariate statistische Modell angewendet wird, das unter Verwendung der folgenden Schritte erzeugt wird:

Bestimmen einer Approximationsfunktion, die einen Satz von klinisch hergeleiteten Referenzmessungen, die Blutdruckparameter repräsentieren, die vom Arterientonus abhängen, wobei die Approximationsfunktion eine Funktion ist von mindestens: (a) einem Parameter, der auf der Standardabweichung der Arteriendruck-Signalform-Daten beruht, (b) einem Parameter, der auf der Herzfrequenz des Probanden beruht, (c) einem Parameter, der auf der Fläche unter dem systolischen Abschnitt des arteriellen Blutdrucksignals beruht, (d) einem Parameter, der auf der Dauer der Systole beruht, (e) einem Parameter, der auf dem Verhältnis der Dauer der Systole zur Dauer der Diastole beruht, (f) einem Parameter, der auf dem mittleren arteriellen Blutdruck eines Satzes von Arteriendruck-Signalform-Daten beruht, (g) einem Parameter, der auf der nach Druck gewichteten Standardabweichung eines Satzes von Arteriendruck-Signalform-Daten beruht, (h) einem Parameter, der auf dem nach Druck gewichteten Mittelwert eines Satzes von Arteriendruck-Signalform-Daten beruht, (i) einem Parameter, der auf den Schiefewerten der arteriellen Pulsschläge eines Satzes von Arteriendruck-Signalform-Daten beruht, (j) einem Parameter, der auf den Kurtosiswerten der arteriellen Pulsschläge eines Satzes von Arteriendruck-Signalform-Daten beruht, (k) einem Parameter, der auf der nach Druck gewichteten Schiefe eines Satzes von Arteriendruck-Signalform-Daten beruht, (l) einem Parameter, der auf der nach Druck gewichteten Kurtosis eines Satzes von Arteriendruck-Signalform-Daten beruht, (m) einem Parameter, der auf der druckabhängigen Windkessel-Compliance eines Satzes von Arteriendruck-Signalform-Daten beruht, und (n) einem Parameter, der auf der Körperoberfläche des Probanden beruht, und einen Satz von klinisch bestimmten Referenzmessungen, die Blutdruckparameter repräsentieren, die vom Arterientonus von Probanden mit normalen hämodynamischen Bedingungen abhängen, verknüpft;

Bestimmen eines Satzes von arteriellen Blutdruck-Parametern aus den arteriellen Blutdruck-Signalform-Daten, wobei der Satz von arteriellen Blutdruck-Parametern mindestens folgendes umfasst: (a) einen Parameter, der auf der Standardabweichung der Arteriendruck-Signalform-Daten beruht, (b) einen Parameter, der auf der Herzfrequenz des Probanden beruht, (c) einen Parameter, der auf der Fläche unter dem systolischen Abschnitt des arteriellen Blutdrucksignals beruht, (d) einen Parameter, der auf der Dauer der Systole beruht, (e) einen Parameter, der auf dem Verhältnis der Dauer der Systole zur Dauer der Diastole beruht, (f) einen Parameter, der auf dem mittleren arteriellen Blutdruck eines Satzes von Arteriendruck-Signalform-Daten beruht, (g) einen Parameter, der auf der nach Druck gewichteten Standardabweichung eines Satzes von Arteriendruck-Signalform-Daten beruht, (h) einen Parameter, der auf dem nach Druck gewichteten Mittelwert eines Satzes von Arteriendruck-Signalform-Daten beruht, (i) einen Parameter, der auf den Schiefewerten der arteriellen Pulsschläge eines Satzes von Arteriendruck-Signalform-Daten beruht, (j) einen Parameter, der auf den Kurtosiswerten der arteriellen Pulsschläge eines Satzes von Arteriendruck-Signalform-Daten beruht, (k) einen Parameter, der auf der nach Druck gewichteten Schiefe eines Satzes von Arteriendruck-Signalform-Daten beruht, (I) einen Parameter, der auf der nach Druck gewichteten Kurtosis eines Satzes von Arteriendruck-Signalform-Daten beruht, (m) einen Parameter, der auf der druckabhängigen Windkessel-Compliance eines Satzes von Arteriendruck-Signalform-Daten beruht, und (n) einen Parameter, der auf der Körperoberfläche des Probanden beruht; und

Berechnen des normalen Arterientonus des Probanden durch Auswerten der Approximationsfunktion mit dem Satz von arteriellen Blutdruck-Parametern.

**Revendications**

1.  Dispositif configuré pour surveiller un découplage de pression artérielle central à périphérique chez un sujet comprenant:

    un capteur (100, 200) pour fournir une donnée de forme d'onde de pression artérielle du sujet;

    un système (300) de traitement pour exécuter un code;

    un module (310) de logiciel comprenant un code pouvant être exécuté par un ordinateur pour appliquer un premier modèle statistique à plusieurs variables à la donnée de forme d'onde de pression artérielle, afin de déterminer le premier tonus artériel du sujet, le modèle statistique à plusieurs variables étant préparé à partir d'un jeu de données de forme d'onde de pression artérielle d'un groupe de sujets de test, qui furent soumis à un découplage de pression artérielle central à périphérique, le premier modèle statistique à plusieurs variables fournissant une valeur du premier tonus artériel du sujet,
    dans lequel le module (310) de logiciel comprend en outre un code pouvant être exécuté par ordinateur pour appliquer un deuxième module statistique à plusieurs variables à la donnée de forme d'onde de pression artérielle, afin de déterminer le tonus artériel normal du sujet, le deuxième module statistique à plusieurs variables étant préparé à partir d'un jeu de données de forme d'onde de pression artérielle d'un groupe de sujets de test ayant des conditions hémodynamiques normales, le deuxième modèle statistique à plusieurs variables fournissant une valeur du deuxième tonus artériel du sujet; et
    un module (320) de calcul comprenant un code exécutable par ordinateur pour comparer le premier tonus artériel du sujet au deuxième tonus artériel du sujet;
    dans lequel le système (300) de traitement détermine un découplage de la pression artérielle centrale à périphérique lorsqu'une différence entre le premier tonus artériel du sujet et le deuxième tonus artériel du sujet plus grande qu'une valeur de seuil indique que le sujet subit un découplage de pression artérielle central à périphérique.

2.  Dispositif configuré pour surveiller un découplage de pression artérielle central à périphérique chez un sujet comprenant:

    un capteur (100, 200) pour fournir une donnée de forme d'onde de pression artérielle du sujet;

    un système (300) de traitement pour exécuter un code;

    un module (310) de logiciel comprenant un code pouvant être exécuté par un ordinateur pour appliquer un premier modèle statistique à plusieurs variables à la donnée de forme d'onde de pression artérielle, afin de déterminer le premier tonus artériel du sujet, le modèle statistique à plusieurs variables étant préparé à partir d'un jeu de données de forme d'onde de pression artérielle d'un groupe de sujets de test, qui furent soumis à un découplage de pression artérielle central à périphérique, le premier modèle statistique à plusieurs variables fournissant une valeur du premier tonus artériel du sujet,
    dans lequel le module (310) de logiciel comprend en outre un code pouvant être exécuté par ordinateur pour appliquer un deuxième module statistique à plusieurs variables à la donnée de forme d'onde de pression artérielle, afin de déterminer le tonus artériel normal du sujet, le deuxième module statistique à plusieurs variables étant préparé à partir d'un jeu de données de forme d'onde de pression artérielle d'un groupe de sujets de test ayant des conditions hémodynamiques normales, le deuxième modèle statistique à plusieurs variables fournissant une valeur du deuxième tonus artériel du sujet; et
    un module (320) de calcul comprenant un code exécutable par ordinateur pour comparer le premier tonus artériel du sujet au deuxième tonus artériel du sujet,
    dans lequel le système (300) de traitement détermine un découplage de pression artérielle centrale à périphérique lorsqu'un rapport du premier tonus artériel du sujet au deuxième tonus artériel du sujet plus grand qu'un rapport de seuil indique que le sujet subit un découplage de pression artérielle central à périphérique.

3. Dispositif suivant l'une des revendications 1 ou 2, le module (320) de calcul étant configuré en outre pour utiliser le premier tonus artériel du sujet et le deuxième tonus artériel du sujet, pour calculer un premier débit cardiaque du sujet et un deuxième débit cardiaque du sujet, une différence entre le premier débit cardiaque du sujet et le deuxième débit cardiaque du sujet plus grande qu'une valeur de seuil indiquant que le sujet subit un découplage de pression artérielle central à périphérique.

4. Dispositif suivant l'une des revendications 1 ou 2, dans lequel le module (320) de calcul est configuré pour calculer la différence entre le premier tonus artériel du sujet et le deuxième tonus artériel du sujet, sous la forme d'un changement en pourcentage du premier tonus artériel du sujet par rapport au deuxième tonus artériel du sujet.

5. Dispositif suivant l'une des revendications 1 ou 2, dans lequel le module statistique à plusieurs variables repose sur un jeu de facteurs comprenant un ou plusieurs paramètres affectés par l'état vasculaire.

6. Dispositif suivant l'une des revendications 1 ou 2, dans lequel le modèle statistique à plusieurs variables repose sur un jeu de facteurs comprenant un ou plusieurs paramètres choisis dans le groupe consistant en (a) un paramètre reposant sur l'écart-type de la donnée de forme d'onde de pression artérielle, (b) un paramètre reposant sur le rythme du coeur, (c) un paramètre reposant sur la surface sous la partie systolique du signal de pression sanguine artérielle, (d) un paramètre reposant sur la durée d'une systole, (e) un paramètre reposant sur le rapport de la durée de la systole à la durée de la diastole, (f) un paramètre reposant sur la pression artérielle moyenne d'un jeu de données de forme d'onde de pression artérielle, (g) un paramètre reposant sur l'écart-type pondéré de pression d'un jeu de données de forme d'onde de pression artérielle, (h) un paramètre reposant sur une moyenne pondérée de pression d'un jeu de données de forme d'onde de pression artérielle, (i) un paramètre reposant sur les valeurs de dissymétrie de battements de pouls artériel d'un jeu de données de forme d'onde de pression artérielle, (j) un paramètre reposant sur les valeurs de curtosis pondérées de pression d'un jeu de données de forme d'onde de pression artérielle, (k) un paramètre reposant sur la dissymétrie pondérée de pression d'un jeu de données de forme d'onde de pression artérielle, (l) un paramètre reposant sur le curtosis pondéré de pression d'un jeu de données de forme d'onde de pression artérielle, (m) un paramètre reposant sur la compliance dépendante de la pression d'un jeu de données de forme d'onde de pression artérielle et (n) un paramètre reposant sur le surface spécifique du corps du sujet.

7. Dispositif suivant l'une des revendications 1 ou 2, dans lequel le modèle statistique à plusieurs variables repose sur un jeu de facteurs comprenant (a) un paramètre reposant sur l'écart-type de la donnée de forme d'onde de pression artérielle, (b) un paramètre reposant sur le rythme cardiaque, (c) un paramètre reposant sur la surface sous la partie systolique du signal de pression sanguine artérielle, (d) un paramètre reposant sur la durée d'une systole, (e) un paramètre reposant sur le rapport de la durée de la systole à la durée de la diastole, (f) un paramètre reposant sur la pression artérielle moyenne d'un jeu de données de forme d'onde de pression artérielle, (g) un paramètre reposant sur l'écart-type pondéré de pression d'un jeu de données de forme d'onde de pression artérielle, (h) un paramètre reposant sur une moyenne pondérée de pression d'un jeu de données de forme d'onde de pression artérielle, (i) un paramètre reposant sur les valeurs de dissymétrie de battements de pouls artériel d'un jeu de données de forme d'onde de pression artérielle, (j) un paramètre reposant sur les valeurs de curtosis pondérées de pression d'un jeu de données de forme d'onde de pression artérielle, (k) un paramètre reposant sur la dissymétrie pondérée de pression d'un jeu de données de forme d'onde de pression artérielle, (l) un paramètre reposant sur le curtosis pondéré de pression d'un jeu de données de forme d'onde de pression artérielle, (m) un paramètre reposant sur la compliance Windkessel dépendante de la pression d'un jeu de données de forme d'onde de pression artérielle et (n) un paramètre reposant sur le surface spécifique du corps du sujet.

8. Dispositif suivant la revendication 1, dans lequel la valeur de seuil est supérieure ou égale à 1 %.

9. Dispositif suivant la revendication 1, comprenant en outre une interface (500) graphique d'utilisateur configurée pour afficher la différence entre le premier tonus artériel du sujet et le deuxième tonus artériel du sujet plus grande que la valeur de seuil.

10. Dispositif suivant la revendication 9, l'interface (500) graphique d'utilisateur étant configurée pour afficher la différence entre le premier tonus artériel du sujet et le deuxième tonus artériel du sujet plus grande que la valeur de seuil, sous la forme d'un graphe à barre ou d'un graphe à tendance.

**11.** Dispositif suivant la revendication 2, comprenant en outre une interface (500) graphique d'utilisateur configurée pour afficher le rapport du premier tonus artériel du sujet au deuxième tonus artériel du sujet plus grand qu'un rapport de seuil.

**12.** Dispositif suivant la revendication 1, comprenant en outre des moyens (330) de traitement pour alerter un utilisateur lorsque la différence entre le premier tonus artériel du sujet et le deuxième tonus artériel du sujet est plus grande que la valeur de seuil.

**13.** Dispositif suivant la revendication 2, comprenant en outre des moyens (330) de traitement pour alerter un utilisateur lorsque le rapport du premier tonus artériel du sujet au deuxième tonus artériel du sujet est plus grand que le rapport de seuil.

**14.** Dispositif suivant l'une des revendications 1 ou 2, dans lequel le module (310) de logiciel est configuré pour déterminer le premier tonus artériel du sujet en appliquant le premier modèle statistique à plusieurs variables créé en utilisant les stades suivants:

on détermine une fonction d'approximation se rapportant à un jeu de mesures de référence obtenues cliniquement, représentant des paramètres de la pression sanguine qui dépendent du tonus artériel, la fonction d'approximation étant une fonction d'au moins (a) un paramètre reposant sur l'écart-type de la donnée de forme d'onde de pression artérielle, (b) un paramètre reposant sur le rythme du coeur, (c) un paramètre reposant sur la surface sous la partie systolique du signal de pression sanguine artérielle, (d) un paramètre reposant sur la durée d'une systole, (e) un paramètre reposant sur le rapport de la durée de la systole à la durée de la diastole, (f) un paramètre reposant sur la pression artérielle moyenne d'un jeu de données de forme d'onde de pression artérielle, (g) un paramètre reposant sur l'écart-type pondéré de pression d'un jeu de données de forme d'onde de pression artérielle, (h) un paramètre reposant sur une moyenne pondérée de pression d'un jeu de données de forme d'onde de pression artérielle, (i) un paramètre reposant sur les valeurs de dissymétrie de battements de pouls artériel d'un jeu de données de forme d'onde de pression artérielle, (j) un paramètre reposant sur les valeurs de curtosis pondérées de pression d'un jeu de données de forme d'onde de pression artérielle, (k) un paramètre reposant sur la dissymétrie pondérée de pression d'un jeu de données de forme d'onde de pression artérielle, (l) un paramètre reposant sur le curtosis pondéré de pression d'un jeu de données de forme d'onde de pression artérielle, (m) un paramètre reposant sur la compliance Windkessel dépendante de la pression d'un jeu de données de forme d'onde de pression artérielle et (n) un paramètre reposant sur le surface spécifique du corps du sujet et un jeu de mesures de référence obtenues cliniquement représentant des paramètres de la pression sanguine dépendant d'un tonus artériel de sujet subissant un découplage de pression artérielle central à périphérique;
on détermine un jeu de paramètres de pression sanguine artérielle à partir de données de forme d'onde de pression sanguine artérielle, le jeu de paramètres de pression sanguine artérielle comprenant au moins (a) un paramètre reposant sur l'écart-type de la donnée de forme d'onde de pression artérielle, (b) un paramètre reposant sur le rythme du coeur, (c) un paramètre reposant sur la surface sous la partie systolique du signal de pression sanguine artérielle, (d) un paramètre reposant sur la durée d'une systole, (e) un paramètre reposant sur le rapport de la durée de la systole à la durée de la diastole, (f) un paramètre reposant sur la pression artérielle moyenne d'un jeu de données de forme d'onde de pression artérielle, (g) un paramètre reposant sur l'écart-type pondéré de pression d'un jeu de données de forme d'onde de pression artérielle, (h) un paramètre reposant sur une moyenne pondérée de pression d'un jeu de données de forme d'onde de pression artérielle, (i) un paramètre reposant sur les valeurs de dissymétrie de battements de pouls artériel d'un jeu de données de forme d'onde de pression artérielle, (j) un paramètre reposant sur les valeurs de curtosis pondérées de pression d'un jeu de données de forme d'onde de pression artérielle, (k) un paramètre reposant sur la dissymétrie pondérée de pression d'un jeu de données de forme d'onde de pression artérielle, (l) un paramètre reposant sur le curtosis pondéré de pression d'un jeu de données de forme d'onde de pression artérielle, (m) un paramètre reposant sur la compliance Windkessel dépendante de la pression d'un jeu de données de forme d'onde de pression artérielle et (n) un paramètre reposant sur le surface spécifique du corps du sujet; et
on estime le premier tonus artériel du sujet en évaluant la fonction d'approximation par le jeu de paramètres de pression sanguine artérielle.

**15.** Dispositif suivant l'une des revendications 1 ou 2, dans lequel le module (310) de logiciel est configuré pour déterminer le tonus artériel normal du sujet en appliquant le deuxième modèle statistique à plusieurs variables en utilisant les stades suivants:

on détermine une fonction d'approximation se rapportant à un jeu de mesures de référence obtenues cliniquement, représentant des paramètres de la pression sanguine qui dépendent du tonus artériel, la fonction d'approximation étant une fonction d'au moins (a) un paramètre reposant sur l'écart-type de la donnée de forme d'onde de pression artérielle, (b) un paramètre reposant sur le rythme du coeur, (c) un paramètre reposant sur la surface sous la partie systolique du signal de pression sanguine artérielle, (d) un paramètre reposant sur la durée d'une systole, (e) un paramètre reposant sur le rapport de la durée de la systole à la durée de la diastole, (f) un paramètre reposant sur la pression artérielle moyenne d'un jeu de données de forme d'onde de pression artérielle, (g) un paramètre reposant sur l'écart-type pondéré de pression d'un jeu de données de forme d'onde de pression artérielle, (h) un paramètre reposant sur une moyenne pondérée de pression d'un jeu de données de forme d'onde de pression artérielle, (i) un paramètre reposant sur les valeurs de dissymétrie de battements de pouls artériel d'un jeu de données de forme d'onde de pression artérielle, (j) un paramètre reposant sur les valeurs de curtosis pondérées de pression d'un jeu de données de forme d'onde de pression artérielle, (k) un paramètre reposant sur la dissymétrie pondérée de pression d'un jeu de données de forme d'onde de pression artérielle, (l) un paramètre reposant sur le curtosis pondéré de pression d'un jeu de données de forme d'onde de pression artérielle, (m) un paramètre reposant sur la compliance Windkessel dépendante de la pression d'un jeu de données de forme d'onde de pression artérielle et (n) un paramètre reposant sur le surface spécifique du corps du sujet et un jeu de mesures de référence obtenues cliniquement représentant des paramètres de la pression sanguine dépendant d'un tonus artériel de sujet ayant des états hémodynamiques normaux;

on détermine un jeu de paramètres de pression sanguine artérielle à partir de données de forme d'onde de pression sanguine artérielle, le jeu de paramètres de pression sanguine artérielle comprenant au moins (a) un paramètre reposant sur l'écart-type de la donnée de forme d'onde de pression artérielle, (b) un paramètre reposant sur le rythme du coeur, (c) un paramètre reposant sur la surface sous la partie systolique du signal de pression sanguine artérielle, (d) un paramètre reposant sur la durée d'une systole, (e) un paramètre reposant sur le rapport de la durée de la systole à la durée de la diastole, (f) un paramètre reposant sur la pression artérielle moyenne d'un jeu de données de forme d'onde de pression artérielle, (g) un paramètre reposant sur l'écart-type pondéré de pression d'un jeu de données de forme d'onde de pression artérielle, (h) un paramètre reposant sur une moyenne pondérée de pression d'un jeu de données de forme d'onde de pression artérielle, (i) un paramètre reposant sur les valeurs de dissymétrie de battements de pouls artériel d'un jeu de données de forme d'onde de pression artérielle, (j) un paramètre reposant sur les valeurs de curtosis pondérées de pression d'un jeu de données de forme d'onde de pression artérielle, (k) un paramètre reposant sur la dissymétrie pondérée de pression d'un jeu de données de forme d'onde de pression artérielle, (l) un paramètre reposant sur le curtosis pondéré de pression d'un jeu de données de forme d'onde de pression artérielle, (m) un paramètre reposant sur la compliance Windkessel dépendante de la pression d'un jeu de données de forme d'onde de pression artérielle et (n) un paramètre reposant sur le surface spécifique du corps du sujet; et

on estime le tonus artériel normal du sujet en évaluant la fonction d'approximation par le jeu de paramètres de pression sanguine artérielle.

*FIG. 1*

*FIG. 2*

**FIG. 3**

**FIG. 4**

**FIG. 5**

**FIG. 6**

**FIG. 7**

**FIG. 8**

**FIG. 9**

**FIG. 10**

FIG. 11

**FIG. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009099833 A1
- US 20080015451 A1
- US 20050124940 A1

**Non-patent literature cited in the description**

- **LANGEWOUTERS et al.** The Static Elastic Properties of 45 Human Thoracic and 20 Abnormal Aortas in vitro and the Parameters of a New Model. *J. Biomechanics,* 1984, vol. 17 (6), 425-435